(19) Europäisches Patentamt
European Patent Office
Office européen des brevets

(11) Numéro de publication : **0 426 998 B1**

(12) # FASCICULE DE BREVET EUROPEEN

(45) Date de publication du fascicule du brevet : **05.05.93 Bulletin 93/18**

(51) Int. Cl.$^5$ : **C07H 17/07**

(21) Numéro de dépôt : **90119380.5**

(22) Date de dépôt : **10.10.90**

(54) **Procédé d'obtention d'isoflavones.**

Demande divisionnaire 91119841.4 déposée le 10/10/90.

(30) Priorité : **10.11.89 CH 4063/89**

(43) Date de publication de la demande : **15.05.91 Bulletin 91/20**

(45) Mention de la délivrance du brevet : **05.05.93 Bulletin 93/18**

(84) Etats contractants désignés : **AT BE CH DE DK ES FR GB GR IT LI LU NL SE**

(56) Documents cités :
**WO-A-80/02098**
**FR-A- 2 542 318**
**US-A- 4 157 984**
**CHEMICAL ABSTRACTS, vol. 108, no. 24, 13 juin 1988, page 366, abrégé n. 210179q, Columbus, Ohio, US; & JP-A-62 126 186 (TSUMURA JUNTENDO INC.) 08-06-1987**

(56) Documents cités :
**CHEMICAL ABSTRACTS, vol. 112, 1990, page 443, abrégé no. 42557y, Columbus, Ohio, US; & JP-A-01 146 894 (TSUMURA & CO.) 08-06-1989**
**AUSTRALIAN JOURNAL OF CHEMISTRY, vol. 24, no. 7, juillet 1971, pages 1509-1518: A.B. BECK et al.: "The acylated isoflavone glycosides from subterranean clover and red clover"**
**J. AGRIC. FOOD. CHEM., vol. 24, no. 6, 1976, pages 1174-1177; M. NAIM et al.: "Antioxidative and antihemolytic activities of soybean isoflavones"**
**Agric. Biol. Chem. 1989, 43; 1415-1419**

(73) Titulaire : **SOCIETE DES PRODUITS NESTLE S.A.**
**Case postale 353**
**CH-1800 Vevey (CH)**

(72) Inventeur : **Fleury, Yvette**
**Impasse de la Côte 4**
**CH-2926 Boncourt (CH)**
Inventeur : **Magnolato, Danièle**
**Av. de la Cressire 5a**
**CH-1814 La Tour-De-Peilz (CH)**

EP 0 426 998 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé d'obtention de deux isoflavones, le malonate de génistine et le malonate de daidzine.

On sait, par exemple par l'article de C.M. Francis (J.Sci. Fd Agric. (1973) 24 1235) que les feuilles de trèfle contiennent du malonate de génistine, de formule:

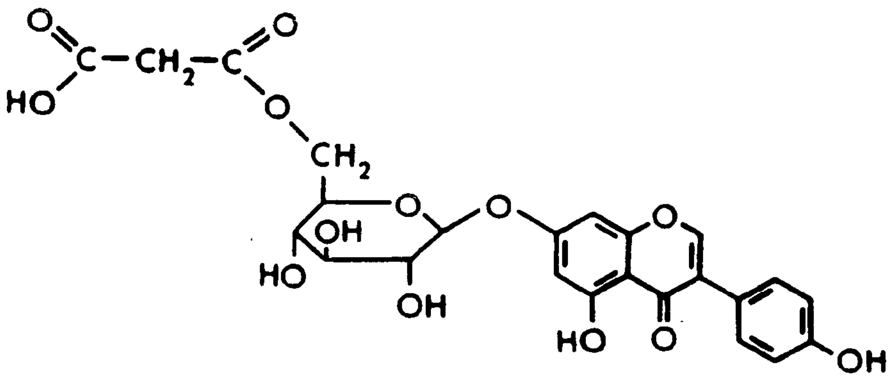

On sait également, par exemple, par le brevet JP 1146894, que l'on peut isoler des racines ou des tiges de Pueraria lobata, le malonate de daidzine, de formule:

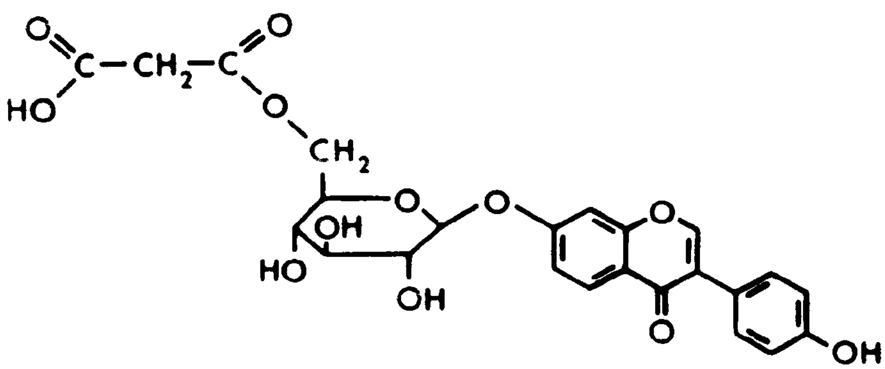

La présente invention a pour but de proposer un procédé permettant l'obtention de ces deux isoflavones particulières à partir d'une source alimentaire, à savoir des fèves de soja, d'une manière simple et économique, et avec un bon rendement.

Le procédé, objet de la présente invention, est caractérisé par le fait que l'on extrait une mouture de fèves de soja avec un alcool, on tamponne l'extrait brut obtenu avec une solution aqueuse de tampon, de manière à atteindre un pH de 6-9, on extrait la solution tampon contenant l'extrait brut avec un solvant organique non miscible à l'eau, on récupère la phase aqueuse que l'on acidifie jusqu'à pH 2,5-4, on extrait la phase aqueuse acidifiée avec un solvant organique non miscible à l'eau, on récupère la phase organique que l'on neutralise à pH 6,8-7,2 et l'on sépare les composés résiduels.

Selon le présent procédé, on peut préparer une mouture de fèves de soja, présentant de préférence un diamètre moyen de l'ordre de 0,5-0,8 mm, et un diamètre maximum de 1,0-1,1 mm.

Préalablement à l'extraction, la mouture de fèves de soja peut être dégraissée, afin d'en ôter les matières grasses contenues dans le soja qui pourraient gêner ultérieurement la bonne mise en oeuvre du procédé. Le dégraissage peut être effectué, par exemple, par chauffage sous reflux de la mouture de fèves de soja dans un solvant organique tel que l'hexane ou l'éther de pétrole.

Dans une forme particulière d'exécution, on peut chauffer un mélange comprenant une partie en poids de mouture de fèves de soja avec 4-6 parties en poids de n-hexane, à une température de 60-70°C, sous une agitation de 70-90 tours par minute et pendant 50-70 minutes, puis laisser refroidir le mélange jusqu'à température ambiante, filtrer puis laver la mouture avec du n-hexane et sécher la mouture dégraissée sous azote pendant 10-15 heures.

On extrait ensuite la mouture éventuellement dégraissée avec un alcool tel que, par exemple le méthanol

ou l'éthanol.

Pour ce faire, on peut, par exemple, chauffer sous reflux un mélange comprenant 1 kg de mouture dégraissée et 6 à 8 litres d'une solution aqueuse de méthanol à 70-90%, à une température de 70-90°C, sous une agitation de 70-90 tours par minute et pendant 50-100 minutes, puis filtrer le mélange à chaud et sécher le filtrat, par exemple dans un évaporateur rotatif à 25-50°C et sous pression réduite.

On peut ainsi obtenir un extrait brut se présentant sous forme d'un solide de couleur brune.

On tamponne ensuite l'extrait brut de manière à amener son pH à une valeur de 6-9.

Pour ce faire, on peut mettre l'extrait brut en suspension ou en solution dans une solution de base faible telle qu'une solution aqueuse de $NaHCO_3$.

Ceci permet d'améliorer l'extraction venant ensuite, la génistine et la daidzine passant dans la phase organique, et les malonates recherchés restant en phase aqueuse.

On effectue alors une extraction de la solution tampon contenant l'extrait brut, avec un solvant organique non miscible à l'eau tel que, par exemple, le butanol ou l'acétate d'éthyle.

Après extraction, on peut constater que la phase organique contient principalement des isoflavones telles que la génistine et la daidzine, ainsi que d'autres constituants, et que la phase aqueuse contient les composés recherchés ainsi que des impuretés.

On acidifie la phase aqueuse jusqu'à un pH de l'ordre de 2,5-4, par exemple par ajout d'acide chlorhydrique concentré, notamment 5-10 N.

On extrait la phase aqueuse acidifiée avec un solvant organique non miscible à l'eau. On peut choisir, de préférence, un solvant identique à celui utilisé pour l'extraction de la suspension ou solution aqueuse contenant l'extrait brut, c'est-à-dire du butanol ou de l'acétate d'éthyle, ce qui permet d'éviter l'emploi, toujours dangereux, de solvants différents.

On peut ensuite neutraliser la phase organique obtenue en amenant son pH à une valeur de 6,8-7,2, par exemple par ajout de soude, puis sécher ladite phase organique, par exemple en évaporant le solvant dans un évaporateur rotatif à 25-50°C et sous pression réduite.

On obtient ainsi un extrait impur, contenant les composés recherchés, se présentant sous forme d'un solide de couleur brune.

On peut ensuite séparer les composés résiduels de cet extrait, par exemple en effectuant une chromatographie d'adsorption et de filtration de l'extrait impur, ce qui permet également d'en séparer les éventuelles impuretés, telles que la génistine et/ou la daidzine encore présentes.

Cette chromatographie peut être effectuée, par exemple, sur une colonne contenant un gel avec comme éluant un alcool tel que l'éthanol ou un mélange méthanol-eau. On peut obtenir plusieurs fractions contenant des impuretés et plusieurs fractions contenant un mélange des composés recherchés.

On peut alors effectuer une seconde chromatographie des fractions intéressantes, afin de séparer les deux composés recherchés.

Pour ce faire, on peut effectuer une chromatographie en phase inversée en utilisant, par exemple, une colonne polaire contenant notamment des groupes octadécylsilane fixés sur de la silice et un éluant, ou un gradient d'éluants de polarité décroissante, tel que, par exemple, une solution à 10-25% de méthanol, d'éthanol ou d'acétonitrile.

On obtient alors deux fractions, contenant chacune un composé distinct.

Ces fractions peuvent être séchées, par exemple dans un évaporateur-à sec, sous pression réduite et à une température de 25-50°C.

On obtient alors deux composés se présentant sous forme de solides amorphes, l'un de couleur jaune pâle, l'autre de couleur blanche, solubles dans des solvants polaires tels que l'eau, le méthanol ou l'éthanol.

On a constaté que ces deux composés présentaient des propriétés antioxydantes remarquables, et qu'ils pouvaient protéger de l'oxydation par exemple les matières grasses, les vitamines et/ou les oligoéléments contenus dans les produits alimentaires ou cosmétiques.

La présente invention est illustrée plus en détails par l'exemple d'obtention qui suit, ainsi que par les tests d'identification des composés et les tests d'activité qui suivent.

Exemple d'obtention des composés

i) Dégraissage

On ajoute 5 kg de n-hexane à 1 kg d'une mouture de fèves de soja, présentant un diamètre moyen de 0,8 mm.

On chauffe le mélange sous reflux à une température de 65°C et sous agitation constante à 80 tours par minute, pendant 1 heure.

On laisse ensuite refroidir le mélange jusqu'à température ambiante puis on filtre et l'on récupère la mouture qui est mise à sécher sous azote pendant 12 heures.
On mélange à nouveau la mouture sèche à 5 kg de n-hexane et l'on chauffe à 65°C, sous reflux et en maintenant une agitation constante, pendant 1 heure.
On laisse refroidir puis on filtre et on lave la mouture résultante avec 1 litre de n-hexane.
On sèche la mouture sous azote pendant 10 heures. On obtient 0,7 kg de mouture dégraissée de fèves de soja.

ii) Préparation de l'extrait brut

On prépare un mélange comprenant 150 g de mouture dégraissée de fèves de soja telle que préparée selon l'étape i), et 1 litre d'une solution aqueuse de méthanol à 80%.
On chauffe le mélange sous reflux à une température de 80°C pendant 1 heure, en maintenant une agitation constante de 80 tours par minute.
On filtre ensuite à chaud le mélange et l'on réserve le filtrat.
On chauffe à nouveau la mouture avec 1 litre de méthanol à 80%, à 80°C, sous agitation et pendant une heure.
On filtre à chaud le mélange et l'on ajoute le filtrat obtenu au filtrat précédent.
On évapore à sec le filtrat total dans un évaporateur rotatif sous pression réduite, à une température de 40°C.
On obtient 32,6 g d'extrait brut se présentant sous forme d'un solide de couleur brune.

iii) Préparation de l'extrait impur

On mélange 32,6 g d'extrait brut tel qu'obtenu selon l'étape ii) à 150 ml d'une solution aqueuse de $NaHCO_3$ 1M.
On ajoute 150 ml de n-butanol, on mélange les deux phases et l'on récupère, par extraction, la phase aqueuse, la phase butanolique contenant principalement de la daidzine et de la génistine.
On acidifie la phase aqueuse jusqu'à pH3 par ajout d'acide chlorhydrique 6,5 N.
On ajoute 150 ml de n-butanol à la phase aqueuse, on mélange et l'on récupère une première phase organique.
On ajoute à nouveau 150 ml de n-butanol à la phase aqueuse, on mélange et l'on récupère une seconde phase organique.
On ajuste le pH des deux phases organiques à 7 par ajout de soude 1N, puis on sèche les deux phases dans un évaporateur rotatif sous pression réduite à 40°C.
On obtient respectivement 2,4 g pour la première phase et 1,2 g pour la seconde phase, d'extrait impur se présentant sous forme d'un solide de couleur brune.

iv) Séparation des composés

On prépare une colonne de chromatographie contenant un gel (Sephadex LH-20), d'un diamètre de 3 cm et d'une hauteur de 50 cm.
On y introduit 2,4 g d'extrait impur tel qu'obtenu selon l'étape iii), dilué avec de l'éthanol.
On élue l'extrait impur avec de l'éthanol, à un débit de 2 ml par minute.
On obtient au total 17 fractions, dont 6 contiennent les malonates de génistine et de daidzine.
On effectue une seconde chromatographie, sur une colonne contenant des groupes octadécylsilane fixés sur de la silice (Lobar RP-18), des 6 fractions intéressantes.
On utilise comme éluant une solution aqueuse d'éthanol à 10%, avec un débit de 2 ml par minute.
On obtient finalement deux fractions contenant chacune un produit différent.
On sèche ces fractions dans un évaporateur sous pression réduite à 40°C.
On obtient 25 mg de malonate de daidzine et 80 mg de malonate de génistine très purs (92-98%) se présentant sous forme de solides amorphes, le premier de couleur blanche et le second de couleur jaune pâle.

Tests d'identification du malonate de génistine

i) Position du goupe sucre

On détermine la position du groupe sucre, de formule:

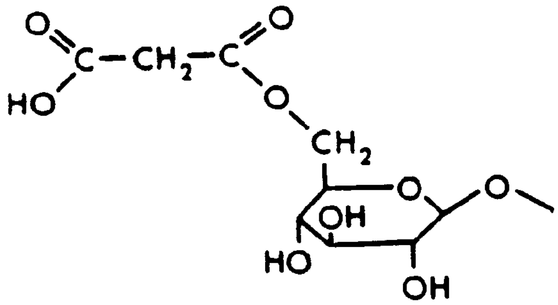

dans le malonate de génistine, à l'aide d'une "shift-reaction" avec du chlorure d'aluminium.

Le groupe OH libre peut se trouver en position 5 ou en position 7, selon la figure ci-après, l'autre position étant occupée par le groupe sucre susmentionné:

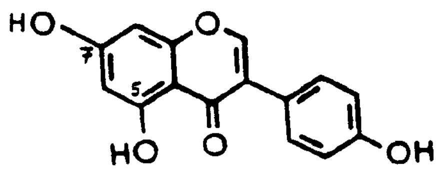

Si le groupe OH se trouve en position 5, il va se produire, en présence de chlorure d'aluminium, la formation d'un complexe selon la réaction:

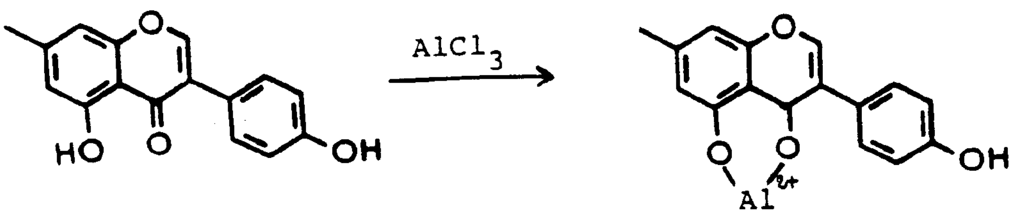


Or le λmax du complexe ainsi formé est supérieur, de 10 à 14 nm environ, au λmax du malonate de génistine. On mesure le λmax du malonate de génistine, dans le méthanol, en présence ou non de chlorure d'aluminium. On obtient les résultats suivants:

. sans $AlCl_3$ λmax = 260,1 nm
. avec $AlCl_3$ λmax = 270,7 nm

Il y a donc eu formation d'un complexe en présence de chlorure d'aluminium; le groupe OH libre est donc en position 5 et le groupe sucre en position 7.

ii) Décomposition en milieu alcalin

On mesure, par chromatographie liquide à haute performance (HPLC) à 228 nm, le temps de rétention de l'acide malonique et du malonate de génistine, tels quels ou en présence d'hydroxyde de sodium.

On observe les résultats suivants:

- acide malonique
  on obtient un pic à 2,19 min
- acide malonique + NaOH
  on obtient deux pics: un à 2,19 min correspondant à l'acide malonique et un à 5,08 min correspondant au malonate de sodium.
- malonate de génistine
  on obtient un pic à 15,17 min
- malonate de génistine + NaOH
  on obtient deux pics: un à 12,80 min correspondant à la génistine et un à 5,07 min correspondant au malonate de sodium.
  Le malonate de génistine s'est donc décomposé, en présence de soude, en malonate de sodium et en génistine.

iii) Spectre infrarouge

On observe sur le spectre IR du malonate de génistine effectué à l'état solide à la concentration de 1% dans KBr, une bande caractéristique à 1729,2 $cm^{-1}$, correspondant au groupement carbonyle de l'ester (valeur théorique 1735 ± 10 $cm^{-1}$).

iv) Spectre de masse

Le spectre de masse a été effectué selon deux méthodes permettant d'obtenir un résultat par défaut ("négatif FAB") et un résultat par excès ("positif FAB").

On obtient les résultats suivants:
- A: pic moléculaire du composé. (malonate de génistine)
- B: pic correspondant au glucoside d'isoflavone (génistine).
- C: pic correspondant à l'aglycone de base (génistéine).

Malonate de génistine (poids moléculaire, g)

| | négatif FAB | positif FAB | valeur théorique |
|---|---|---|---|
| A | 517 | 519 | 518 |
| B | 431 | 433 | 432 |
| C | 269 | 271 | 270 |

v) Spectre RMN

Le spectre de résonance magnétique nucléaire du carbone 13 (RMN) du malonate de génistine dans le diméthylsulfoxyde (DMSO-d6) à 20°C montre les signaux caractéristiques suivants:

| Signal | Multiplicité |
|---|---|
| 45,4 ppm | $CH_2$ |
| 63,1 ppm | $CH_2$ |
| 69,7 ppm | CH |
| 73,0 ppm | CH |
| 74,0 ppm | CH |
| 76,0 ppm | CH |
| 94,3 ppm | CH |
| 99,4 ppm | CH |
| 99,7 ppm | CH |
| 106,3 ppm | C |
| 115,0 ppm | CH (2 carbones) |
| 120,8 ppm | C |
| 122,6 ppm | C |
| 130,0 ppm | CH (2 carbones) |
| 154,0 ppm | CH |
| 157,2 ppm | C |
| 157,7 ppm | C |
| 161,7 ppm | C |
| 162,5 ppm | C |
| 168,4 ppm | C |
| 169,2 ppm | C |
| 179,9 ppm | C |

On trouve ainsi 24 atomes de carbone, parmi lesquels 3 carbones carbonyles (acide carboxylique et cétone conjuguée), 14 carbones aromatiques ou oléfiniques (7CH, 7 quaternaires) dont 5 sont O-substitués, un carbone anomérique CH-OH, 4 CH aliphatiques O-substitués, un $CH_2$ aliphatique O-substitué et un $CH_2$ aliphatique non O-substitué.
On trouve également 16 protons non interchangeables.

Tests d'identification du malonate de daidzine

i) Décomposition en milieu alcalin

On mesure, par chromatographie liquide à haute performance (HPLC) à 228 nm, le temps de rétention de l'acide malonique et du malonate de daidzine, tels quels ou en présence d'hydroxyde de sodium.
On observe les résultats suivants:
- acide malonique
  on obtient un pic à 2,19 min
- acide malonique + NaOH
  on obtient deux pics: un à 2,19 min correspondant à l'acide malonique et un à 5,08 min correspondant au malonate de sodium.
- daidzine + NaOH
  on obtient un pic à 8,66 min

- malonate de daidzine
  on obtient un pic à 13,89 min
- malonate de daidzine + NaOH

on obtient:

a) immédiatement après le mélange, un pic à 11,47 min correspondant à la daidzine et un pic à 8,65 min correspondant à celui obtenu pour la daidzine en présence de NaOH,

b) une heure après le mélange, un pic à 8,65 min correspondant à celui obtenu pour la daidzine en présence de NaOH.

ii) Spectre infrarouge

On observe sur le spectre IR du malonate de daidzine effectué à l'état solide à la concentration de 1% dans KBr, une bande caractéristique à 1734,0 $cm^{-1}$, correspondant au groupement carbonyle de l'ester (valeur théorique 1735 ± 10 $cm^{-1}$).

iii) Spectre de masse

Un spectre de masse a été effectué selon deux méthodes permettant d'obtenir un résultat par défaut ("négatif FAB") et un résultant par excès ("positif FAB").

On obtient les résultats suivants:

- A : pic moléculaire du composé (malonate de daidzine).
- B: pic correspondant au glucoside d'isoflavone (daidzine).
- C: pic correspondant à l'aglycone de base (daidzéine)

Malonate de daidzine (poids moléculaire, g)

| | négatif FAB | positif FAB | valeur théorique |
|---|---|---|---|
| A | 501 | 503 | 502 |
| B | 415 | 417 | 416 |
| C | 253 | 255 | 254 |

iv) Spectre RMN

Le spectre de résonance magnétique nucléaire du carbone 13 (RMN) du malonate de daidzine dans le diméthylsulfoxyde (DMSO-d6) à 20°C montre les signaux caractéristiques suivants:

| Signal | Multiplicité |
|---|---|
| 45,2 ppm | $CH_2$ |
| 63,0 ppm | $CH_2$ |
| 69,7 ppm | CH |
| 73,0 ppm | CH |
| 74,0 ppm | CH |
| 76,1 ppm | CH |
| 99,7 ppm | CH |
| 103,4 ppm | CH |
| 114,9 ppm | CH (2 carbones) |
| 115,3 ppm | CH |
| 118,4 ppm | C |
| 122,0 ppm | C |
| 123,6 ppm | C |
| 126,9 ppm | CH |
| 129,9 ppm | CH (2 carbones) |
| 153,2 ppm | CH |
| 156,8 ppm | C |
| 157,4 ppm | C |
| 161,0 ppm | C |
| 168,3 ppm | C |
| 169,1 ppm | C |
| 174,7 ppm | C |

On trouve donc un total de 24 atomes de carbone, parmi lesquels 3 carbones carbonyles (acide carboxylique et cétone conjuguée), 14 carbones aromatiques ou oléfiniques (8CH, 6 quaternaires) dont 4 sont O-substitués, un carbone est anomérique (CH-OH), 4 sont des CH aliphatiques O-substitués, un est un $CH_2$ aliphatique O-substitué et un est un $CH_2$ aliphatique non O-substitué.
On a également trouvé 17 protons non interchangeables.

Tests d'activité des composés

i) Analyse qualitative

Il est connu que le β carotène est oxydé en présence d'acide linoléique par irradiation aux ultraviolets (UV). L'oxydation est mise en évidence par la décoloration du β carotène.

On dépose sur une plaque support pour chromatographie sur couche mince, une goutte d'une solution aqueuse de daidzine, de génistine, de malonate de daidzine ou de malonate de génistine.
On vaporise ensuite sur la plaque une solution contenant 100 mg de β carotène et 1 ml d'acide linoléique dans 100 ml de chloroforme. La plaque devient jaune.
On place la plaque sous une source de lumière UV et on l'y laisse jusqu'à l'apparition d'une décoloration.
On observe alors qu'aux endroits où l'on a déposé la génistine et la daidzine, la plaque n'est pas décolorée et présente toujours une coloration jaune.
Ceci est dû au fait que la daidzine et la génistine présentent des propriétés antioxydantes qui peuvent empêcher l'oxydation du β carotène, donc sa décoloration.
On observe aussi que la plaque présente la même coloration jaune aux endroits où l'on a déposé les malonates de génistine et de daidzine.
Ces deux composés présentent donc aussi des propriétés antioxydantes.

ii) Analyse quantitative par spectrophotométrie

On prépare des solutions 0,01 M dans le méthanol de daidzéine, de génistéine, de daidzine, de génistine, de malonate de daidzine et de malonate de génistine.

De manière identique, on prépare code étalons plusieurs solutions d'acide gallique dans le méthanol, dont la concentration varie de 0,2 à 20 $gl^{-1}$.

On prépare également, comme réactif, une solution contenant 100 mg de β carotène, 1 ml d'acide linoléique et 100 ml de chloroforme.

On prépare des premiers échantillons contenant 20 µl de la solution à mesurer, 60 µl de réactif et 2 ml de méthanol, et des seconds échantillons témoins contenant 20 µl de la solution à mesurer et 2 ml de méthanol.

On mesure, à 450 nm, l'absorbance de l'échantillon par rapport à son échantillon témoin juste après leurs préparations, puis l'on expose les deux échantillons pendant 12 minutes à un rayonnement ultraviolet et l'on effectue à nouveau la mesure de leur absorbance.

On peut alors comparer les résultats obtenus pour les isoflavones aux résultats obtenus pour les différentes solutions d'acide gallique, ce qui permet de définir les équivalences suivantes:

| Solution d'isoflavones 0,010 M | Concentration molaire de la solution d'acide gallique présentant le même pouvoir antioxydant |
|---|---|
| Daidzéine | 0,020 M |
| Daidzine | 0,022 M |
| Malonate de daidzine | 0,024 M |
| Génistéine | 0,028 M |
| Génistine | 0,027 M |
| Malonate de génistine | 0,029 M |

On constate donc que les solutions 0,010 M de malonate de daidzine ou de génistine ont le même effet que les solutions 0,010 M de daidzine ou de génistine, et que les solutions 0,024 M et 0,029 M d'acide gallique.

iii) Test d'oxydation

En utilisant le test d'oxydation accélérée Rancimat, on détermine les temps d'induction de la graisse de poule stabilisée par addition de malonate de génistine ou de daidzine.

Le test Rancimat® consiste à faire passer de l'air dans un tube de réaction contenant un échantillon de 5 g de matière grasse à 100°C et à mesurer la conductivité des produits secondaires volatils formés en cours d'oxydation et entraînés avec le courant d'air. On détermine le temps d'induction graphiquement à partir de la courbe enregistrée de la conductivité en fonction du temps par intersection de la tangente à la courbe avec l'axe des temps.

On obtient les résultats suivants pour une graisse de poule contenant 500 ppm de malonate de génistine ou de daidzine ou ne contenant rien (témoin) :

| | | temps d'induction (h) |
|---|---|---|
| témoin | | 6,95 |
| malonate de génistine | 500 ppm | 8,10 |
| malonate de daidzine | 500 ppm | 7,68 |

L'effet antioxydant du malonate de génistine et du malonate de daidzine est donc bien confirmé.

## Revendications

1. Procédé d'obtention d'isoflavones caractérisé par le fait que l'on extrait une mouture de fèves de soja avec un alcool, on tamponne l'extrait brut obtenu avec une solution aqueuse de tampon, de manière à atteindre un pH de 6-9, on extrait la solution tampon contenant l'extrait brut avec un solvant organique non miscible à l'eau, on récupère la phase aqueuse que l'on acidifie jusqu'à pH 2,5-4, on extrait la phase aqueuse acidifiée avec un solvant organique non miscible à l'eau, on récupère la phase organique que l'on neutralise à pH 6,8 -7,2 et l'on sépare les composés résiduels.

2. Procédé selon la revendication 1, caractérisé par le fait que l'on extrait la mouture de fèves de soja avec une solution aqueuse de méthanol à 70-90%, par chauffage à 70-90°C pendant 50-100 minutes.

3. Procédé selon la revendication 1, caractérisé par le fait que le solvant organique non miscible à l'eau est le butanol.

## Claims

1. A process for the production of isoflavones, characterized in that ground soya beans are extracted with an alcohol, the crude extract obtained is buffered with an aqueous buffer solution to a pH value of 6 - 9, the buffer solution containing the crude extract is extracted with a water-immiscible organic solvent, the aqueous phase is recovered and acidified to pH 2.5 - 4, the acidified aqueous phase is extracted with a water-immiscible organic solvent, the organic phase is recovered and neutralized to pH 6.8 - 7.2 and the residual constituents are separated.

2. A process as claimed in claim 1, characterized in that the ground soya beans are extracted with a 70 - 90% aqueous methanol solution by heating for 50 - 100 minutes at 70 - 90°C.

3. A process as claimed in claim 1, characterized in that the water-immiscible organic solvent is butanol.

## Patentansprüche

1. Verfahren zur Herstellung von Isoflavonen, dadurch gekennzeichnet, daß man ein Sojabohnenmehl mit einem Alkohol extrahiert, den erhaltenen Rohextrakt mit einer wässrigen Pufferlösung puffert, so daß man einenq pH von 6 bis 9 erreicht, die den Rohextrakt enthaltende Pufferlösung mit einem nicht mit Wasser mischbaren organischen Lösungsmittel extrahiert, die wässrige Phase gewinnt, die bis zu pH 2,5 bis 4 angesäuert wird, die angesäuerte wässrige Phase mit einem nicht mit Wasser mischbaren organischen Lösungsmittel extrahiert, die organische Phase gewinnt, die auf pH 6,8 bis 7,2 neutralisiert wird, und die zurückbleibenden Verbindungen auftrennt.

2. Verfahren nach Anspruch 1, dadurch gekennzeichnet, daß man das Sojabohnenmehl mit einer 70 bis 90%-igen wässrigen Methanollösung durch Erhitzen auf 70 bis 90°C während 50 bis 100 Minuten extrahiert.

3. Verfahren nach Anspruch 1, dadurch gekennzeichnet, daß das nicht mit Wasser mischbare organische Lösungsmittel Butanol ist.